# EUROPEAN PATENT APPLICATION

(11) **EP 0 832 972 A2**
(43) Date of publication of application: **01.04.1998**
(21) Application number: 97116132.8
(22) Date of filing: 17.09.1997
(51) Int. Cl.: C12N 15/12

(54) **Cloning, expression and production of tasty peptides**

(30) Priority: 23.09.1996 EP 96115211
(71) Applicant: GIVAUDAN-ROURE (INTERNATIONAL) S.A., 1214 Vernier, Genève (CH)
(72) Inventor: Lerch, Konrad, 8118 Pfaffhausen (CH); Muheim, Andreas, 8002 Zurich (CH); Silke, Natasha, Carlton, Victoria 3053 (AU)
(74) Representative: Buntz, Gerhard

(57) **Abstract**

The present invention relates to organisms comprising tasty peptides or parts or extracts thereof, the production of tasty peptides in organisms via recombinant expression of said tasty peptides. Organisms comprising tasty peptides or parts or extracts thereof, tasty peptides in homogeneous form, DNA sequences encoding these peptides and vectors containing these DNA sequences are provided, along with methods for recombinantly producing such peptides, DNA sequences, vectors and organisms.

SEQUENCE LISTING

(1) GENERAL INFORMATION:

(i) APPLICANT:
(A) NAME: GIVAUDAN-ROURE (INTERNATIONAL) SA
(B) STREET: CH. PARFUMERIE 5
(C) CITY: VERNIER
(D) STATE: GENEVA
(E) COUNTRY: SWITZERLAND
(F) POSTAL CODE (ZIP): CH-1214
(G) TELEPHONE: 061-688 13 95
(H) TELEFAX: 061-688 13 95
(I) TELEX: 962292/965542

(ii) TITLE OF INVENTION: CLONING, EXPRESSION AND PRODUCTION OF TASTY
PEPTIDES

(iii) NUMBER OF SEQUENCES: 23

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97116132.8

(2) INFORMATION FOR SEQ ID NO: 1:

(i) SEQUENCE CHARACTERISTICS:
(A) LENGTH: 8 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 1:

Lys Gly Asp Glu Glu Ser Leu Ala
1               5

(2) INFORMATION FOR SEQ ID NO: 2:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 2:

Asp Lys Ile His Pro Phe
1               5

(2) INFORMATION FOR SEQ ID NO: 3:

(i) SEQUENCE CHARACTERISTICS:
(A) LENGTH: 7 amino acids

(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 3:

Arg Gly Pro Phe Ile Ile Val
1               5

(2) INFORMATION FOR SEQ ID NO: 4:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "SYNTHETIC DNA"

(viii) POSITION IN GENOME:
(C) UNITS: bp

(xi) SEQUENCE DESCRIPTION: SEQ ID NO: 4:

AAGGGTGACG AAGAATCTTT GGCTTAGA                                    28

(2) INFORMATION FOR SEQ ID NO: 5:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "SYNTHETIC DNA"

(xi) SEQUENCE DESCRIPTION: SEQ ID NO: 5:

AGCTTCTAAG CCAAAGATTC TTCGTCACCC TT                               32

(2) INFORMATION FOR SEQ ID NO: 6:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "SYNTHETIC DNA"

(xi) SEQUENCE DESCRIPTION: SEQ ID NO: 6:

TCGAGAAGAG AGAAGCTGAA GCTAAGGGT                                   29

(2) INFORMATION FOR SEQ ID NO: 7:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 7:

CGTCACCCTT AGCTTCAGCT TCTCTCTTC 29

(2) INFORMATION FOR SEQ ID NO: 8:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 8:

GACGAAGAAT CTTTGGCTTG AG 22

(2) INFORMATION FOR SEQ ID NO: 9:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 9:

GATCCTCAAG CCAAAGATTC TT 22

(2) INFORMATION FOR SEQ ID NO: 10:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 10:

GACGAAGAAT CTTTGGCTAA GAGAGAAGCT GAAGCTAAGG GT 42

(2) INFORMATION FOR SEQ ID NO: 11:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 11:

TTCTTAGAAA CCGATTCTCT CTTCGACTTC GATTCCCACT GC 42

(2) INFORMATION FOR SEQ ID NO: 12:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 12:

TAGACTGGCG TTGTAATG 18

(2) INFORMATION FOR SEQ ID NO: 13:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 13:

GACGAAGAAT CTTTGGCTAA GGGT 24

(2) INFORMATION FOR SEQ ID NO: 14:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 14:

CGTCACCCTT AGCCAAAGAT TCTT 24

(2) INFORMATION FOR SEQ ID NO: 15:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "CYNTHETIC DNA"

(xi) SEQUENCE DESCRIPTION: SEQ ID NO: 15:

TCGAGAAGAG AGAAGCTGAA GCTAAGGGT 29

(2) INFORMATION FOR SEQ ID NO: 16:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 16:

CGTCACCCTT AGCTTCAGCT TCTCTCTTC 29

(2) INFORMATION FOR SEQ ID NO: 17:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 17:

GACGAAGAAT CTTTGGCTTG AG 22

(2) INFORMATION FOR SEQ ID NO: 18:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 18:

GATCCTCAAG CCAAAGATTC TT 22

(2) INFORMATION FOR SEQ ID NO: 19:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 19:

TCGAGAAGAG AGAAGCTGAA GCTGACAAGA TTCACCCATT CTGAG 45

(2) INFORMATION FOR SEQ ID NO: 20:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 20:

GATCCTCAGA ATGGGTGAAT CTTGTCAGCT TCAGCTTCTC TCTTC 45

(2) INFORMATION FOR SEQ ID NO: 21:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 21:

TCGAGAAGAG AGAAGCTGAA GCTAGAGGTC CATTCCCAAT TATTGTTTGA G 51

(2) INFORMATION FOR SEQ ID NO: 22:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "SYNTHETIC DNA"

(xi) SEQUENCE DESCRIPTION: SEQ ID NO: 22:

GATCCTCAAA CAATAATTGG GAATGGACCT CTAGCTTCAG CTTCTCTCTT C 51

(2) INFORMATION FOR SEQ ID NO: 23:

(i) SEQUENCE CHARACTERISTICS:
(A) LENGTH: 8140 base pairs
(B) TYPE: nucleic acid
(C) STRANDEDNESS: double
(D) TOPOLOGY: circular

(ii) MOLECULE TYPE: DNA (genomic)

(vi) ORIGINAL SOURCE:
(A) ORGANISM: PLASMID PNS2

(xi) SEQUENCE DESCRIPTION: SEQ ID NO: 23:

CCGATGCCCT TGAGAGCCTT CAACCCAGTC AGCTCCTTCC GGTGGGCGCG GGGCATGACT 60

ATCGTCGCCG CACTTATGAC TGTCTTCTTT ATCATGCAAC TCGTAGGACA GGTGCCGGCA 120

GCGCTCTGGG TCATTTTCGG CGAGGACCGC TTTCGCTGGA GCGCGACGAT GATCGGCCTG 180

TCGCTTGCGG TATTCGGAAT CTTGCACGCC CTCGCTCAAG CCTTCGTCAC TGGTCCCGCC 240

ACCAAACGTT TCGGCGAGAA GCAGGCCATT ATCGCCGGCA TGGCGGCCGA CGCGCTGGGC 300

TACGTCTTGC TGGCGTTCGC GACGCGAGGC TGGATGGCCT TCCCCATTAT GATTCTTCTC 360

GCTTCCGGCG GCATCGGGAT GCCCGCGTTG CAGGCCATGC TGTCCAGGCA GGTAGATGAC 420

GACCATCAGG GACAGCTTCA AGGATCGCTC GCGGCTCTTA CCAGCCTAAC TTCGATCACT 480

GGACCGCTGA TCGTCACGGC GATTTATGCC GCCTCGGCGA GCACATGGAA CGGGTTGGCA 540

TGGATTGTAG GCGCCGCCCT ATACCTTGTC TGCCTCCCCG CGTTGCGTCG CGGTGCATGG 600

AGCCGGGCCA CCTCGACCTG AATGGAAGCC GGCGGCACCT CGCTAACGGA TTCACCACTC 660

CAAGAATTGG AGCCAATCAA TTCTTGCGGA GAACTGTGAA TGCGCAAACC AACCCTTGGC 720

AGAACATATC CATCGCGTCC GCCATCTCCA GCAGCCGCAC GCGGCGCATC TCGGAGCTTT 780

TCAATTCAAT TCATCATTTT TTTTTTATTC TTTTTTTTGA TTTCGGTTTC TTTGAAATTT 840

TTTTGATTCG GTAATCTCCG AACAGAAGGA AGAACGAAGG AAGGAGCACA GACTTAGATT 900

GGTATATATA CGCATATGTA GTGTTGAAGA AACATGAAAT TGCCCAGTAT TCTTAACCCA 960

ACTGCACAGA ACAAAAACCT GCAGGAAACG AAGATAAATC ATGTCGAAAG CTACATATAA 1020

GGAACGTGCT GCTACTCATC CTAGTCCTGT TGCTGCCAAG CTATTTAATA TCATGCACGA 1080

AAAGCAAACA AACTTGTGTG CTTCATTGGA TGTTCGTACC ACCAAGGAAT TACTGGAGTT 1140

AGTTGAAGCA TTAGGTCCCA AAATTTGTTT ACTAAAAACA CATGTGGATA TCTTGACTGA 1200

TTTTTCCATG GAGGGCACAG TTAAGCCGCT AAAGGCATTA TCCGCCAAGT ACAATTTTTT 1260

ACTCTTCGAA GACAGAAAAT TTGCTGACAT TGGTAATACA GTCAAATTGC AGTACTCTGC 1320

GTGACTGGGA AAACCCTGGC GTTACCCAAC TTAATCGCCT TGCAGCACAT CCCCCTTTCG 3480
CCAGCTGGCG TAATAGCGAA GAGGCCCGCA CCGATCGCCC TTCCCAACAG TTGCGCAGCC 3540
TGAATGGCGA ATGGCGCGGG AATCTCGGTC GTAATGATTT TTATAATGAC GAAAAAAAAA 3600
AAATTGGAAA GAAAAAGCTT CGGGCAGCGT TGGGTCCTGG CCACGGGTGC GCATGATCGT 3660
GCTCCTGTCG TTGAGGACCC GGCTAGGCTG GCGGGGTTGC CTTACTGGTT AGCAGAATGA 3720
ATCACCGATA CGCGAGCGAA CGTGAAGCGA CTGCTGCTGC AAAACGTCTG CGACCTGAGC 3780
AACAACATGA ATGGTCTTCG GTTTCCGTGT TTCGTAAAGT CTGGAAACGC GGAAGTCAGC 3840
GCCCTGCACC ATTATGTTCC GGATCTGCAT CGCAGGATGC TGCTGGCTAC CCTGTGGAAC 3900
ACCTACATCT GTATTAACGA AGCGCTGGCA TTGACCCTGA GTGATTTTTC TCTGGTCCCG 3960
CCGCATCCAT ACCGCCAGTT GTTTACCCTC ACAACGTTCC AGTAACCGGG CATGTTCATC 4020
ATCAGTAACC CGTATCGTGA GCATCCTCTC TCGTTTCATC GGTATCATTA CCCCCATGAA 4080
CAGAAATTCC CCCTTACACG GAGGCATCAA GTGACCAAAC AGGAAAAAAC CGCCCTTAAC 4140
ATGGCCCGCT TTATCAGAAG CCAGACATTA ACGCTTCTGG AGAAACTCAA CGAGCTGGAC 4200
GCGGATGAAC AGGCAGACAT CTGTGAATCG CTTCACGACC ACGCTGATGA GCTTTACCGC 4260
AGAGCTTTGA AGAAAAATGC GCCTTATTCA ATCTTTGCTA TAAAAAATGG CCCAAAATCT 4320
CACATTGGAA GACATTTGAT GACCTCATTT CTTTCAATGA AGGGCCTAAC GGAGTTGACT 4380
AATGTTGTGG GAAATTGGAG CGATAAGCGT GCTTCTGCCG TGGCCAGGAC AACGTATACT 4440
CATCAGATAA CAGCAATACC TGATCACTAC TTCGCACTAG TTTCTCGGTA CTATGCATAT 4500
GATCCAATAT CAAAGGAAAT GATAGCATTG AAGGATGAGA CTAATCCAAT TGAGGAGTGG 4560
CAGCATATAG AACAGCTAAA GGGTAGTGCT GAAGGAAGCA TACGATACCC CGCATGGAAT 4620
GGGATAATAT CACAGGAGGT ACTAGACTAC CTTTCATCCT ACATAAATAG ACGCATATAA 4680
GTACGCATTT AAGCATAAAC ACGCACTATG CCGTTCTTCT CATGTATATA TATATACAGG 4740
CAACACGCAG ATATAGGTGC GACGTGAACA GTGAGCTGTA TGTGCGCAGC TCGCGTTGCA 4800
TTTTCGGAAG CGCTCGTTTT CGGAAACGCT TTGAAGTTCC TATTCCGAAG TTCCTATTCT 4860
CTAGCTAGAA AGTATAGGAA CTTCAGAGCG CTTTTGAAAA CCAAAAGCGC TCTGAAGACG 4920
CACTTTCAAA AAACCAAAAA CGCACCGGAC TGTAACGAGC TACTAAAATA TTGCGAATAC 4980
CGCTTCCACA AACATTGCTC AAAAGTATCT CTTTGCTATA TATCTCTGTG CTATATCCCT 5040
ATATAACCTA CCCATCCACC TTTCGCTCCT TGAACTTGCA TCTAAACTCG ACCTCTACAT 5100
TTTTTATGTT TATCTCTAGT ATTACTCTTT AGACAAAAAA ATTGTAGTAA GAACTATTCA 5160
TAGAGTGAAT CGAAAACAAT ACGAAAATGT AAACATTTCC TATACGTAGT ATATAGAGAC 5220
AAAATAGAAG AAACCGTTCA TAATTTTCTG ACCAATGAAG AATCATCAAC GCTATCACTT 5280
TCTGTTCACA AAGTATGCGC AATCCACATC GGTATAGAAT ATAATCGGGG ATGCCTTTAT 5340
CTTGAAAAAA TGCACCCGCA GCTTCGCTAG TAATCAGTAA ACGCGGGAAG TGGAGTCAGG 5400
CTTTTTTTAT GGAAGAGAAA ATAGACACCA AAGTAGCCTT CTTCTAACCT TAACGGACCT 5460
ACAGTGCAAA AAGTTATCAA GAGACTGCAT TATAGAGCGC ACAAAGGAGA AAAAAAGTAA 5520

TCTAAGATGC TTTGTTAGAA AAATAGCGCT CTCGGGATGC ATTTTTGTAG AACAAAAAAG 5580
AAGTATAGAT TCTTTGTTGG TAAAATAGCG CTCTCGCGTT GCATTTCTGT TCTGTAAAAA 5640
TGCAGCTCAG ATTCTTTGTT TGAAAAATTA GCGCTCTCGC GTTGCATTTT TGTTTTACAA 5700
AAATGAAGCA CAGATTCTTC GTTGGTAAAA TAGCGCTTTC GCGTTGCATT TCTGTTCTGT 5760
AAAAATGCAG CTCAGATTCT TTGTTTGAAA AATTAGCGCT CTCGCGTTGC ATTTTTGTTC 5820
TACAAAATGA AGCACAGATG CTTCGTTCTG CCTCGCGCGT TTCGGTGATG ACGGTGAAAA 5880
CCTCTGACAC ATGCAGCTCC CGGAGACGGT CACAGCTTGT CTGTAAGCGG ATGCCGGGAG 5940
CAGACAAGCC CGTCAGGGCG CGTCAGCGGG TGTTGGCGGG TGTCGGGGCG CAGCCATGAC 6000
CCAGTCACGT AGCGATAGCG GAGTGTATAC TGGCTTAACT ATGCGGCATC AGAGCAGATT 6060
GTACTGAGAG TGCACCATAT GCGGTGTGAA ATACCGCACA GATGCGTAAG GAGAAAATAC 6120
CGCATCAGGC GCTCTTCCGC TTCCTCGCTC ACTGACTCGC TGCGCTCGGT CGTTCGGCTG 6180
CGGCGAGCGG TATCAGCTCA CTCAAAGGCG GTAATACGGT TATCCACAGA ATCAGGGGAT 6240
AACGCAGGAA AGAACATGTG AGCAAAAGGC CAGCAAAAGG CCAGGAACCG TAAAAAGGCC 6300
GCGTTGCTGG CGTTTTTCCA TAGGCTCCGC CCCCCTGACG AGCATCACAA AAATCGACGC 6360
TCAAGTCAGA GGTGGCGAAA CCCGACAGGA CTATAAAGAT ACCAGGCGTT TCCCCCTGGA 6420
AGCTCCCTCG TGCGCTCTCC TGTTCCGACC CTGCCGCTTA CCGGATACCT GTCCGCCTTT 6480
CTCCCTTCGG GAAGCGTGGC GCTTTCTCAT AGCTCACGCT GTAGGTATCT CAGTTCGGTG 6540
TAGGTCGTTC GCTCCAAGCT GGGCTGTGTG CACGAACCCC CCGTTCAGCC CGACCGCTGC 6600
GCCTTATCCG GTAACTATCG TCTTGAGTCC AACCCGGTAA GACACGACTT ATCGCCACTG 6660
GCAGCAGCCA CTGGTAACAG GATTAGCAGA GCGAGGTATG TAGGCGGTGC TACAGAGTTC 6720
TTGAAGTGGT GGCCTAACTA CGGCTACACT AGAAGGACAG TATTTGGTAT CTGCGCTCTG 6780
CTGAAGCCAG TTACCTTCGG AAAAAGAGTT GGTAGCTCTT GATCCGGCAA ACAAACCACC 6840
GCTGGTAGCG GTGGTTTTTT TGTTTGCAAG CAGCAGATTA CGCGCAGAAA AAAAGGATCT 6900
CAAGAAGATC CTTTGATCTT TTCTACGGGG TCTGACGCTC AGTGGAACGA AAACTCACGT 6960
TAAGGGATTT TGGTCATGAG ATTATCAAAA AGGATCTTCA CCTAGATCCT TTTAAATTAA 7020
AAATGAAGTT TTAAATCAAT CTAAAGTATA TATGAGTAAA CTTGGTCTGA CAGTTACCAA 7080
TGCTTAATCA GTGAGGCACC TATCTCAGCG ATCTGTCTAT TTCGTTCATC CATAGTTGCC 7140
TGACTCCCCG TCGTGTAGAT AACTACGATA CGGGAGGGCT TACCATCTGG CCCCAGTGCT 7200
GCAATGATAC CGCGAGACCC ACGCTCACCG GCTCCAGATT TATCAGCAAT AAACCAGCCA 7260
GCCGGAAGGG CCGAGCGCAG AAGTGGTCCT GCAACTTTAT CCGCCTCCAT CCAGTCTATT 7320
AATTGTTGCC GGGAAGCTAG AGTAAGTAGT TCGCCAGTTA ATAGTTTGCG CAACGTTGTT 7380
GCCATTGCTG CAGGCATCGT GGTGTCACGC TCGTCGTTTG GTATGGCTTC ATTCAGCTCC 7440
GGTTCCCAAC GATCAAGGCG AGTTACATGA TCCCCCATGT TGTGCAAAAA AGCGGTTAGC 7500

TCCTTCGGTC CTCCGATCGT TGTCAGAAGT AAGTTGGCCG CAGTGTTATC ACTCATGGTT 7560
ATGGCAGCAC TGCATAATTC TCTTACTGTC ATGCCATCCG TAAGATGCTT TTCTGTGACT 7620
GGTGAGTACT CAACCAAGTC ATTCTGAGAA TAGTGTATGC GGCGACCGAG TTGCTCTTGC 7680
CCGGCGTCAA CACGGGATAA TACCGCGCCA CATAGCAGAA CTTTAAAAGT GCTCATCATT 7740
GGAAAACGTT CTTCGGGGCG AAAACTCTCA AGGATCTTAC CGCTGTTGAG ATCCAGTTCG 7800
ATGTAACCCA CTCGTGCACC CAACTGATCT TCAGCATCTT TTACTTTCAC CAGCGTTTCT 7860
GGGTGAGCAA AAACAGGAAG GCAAAATGCC GCAAAAAAGG GAATAAGGGC GACACGGAAA 7920
TGTTGAATAC TCATACTCTT CCTTTTTCAA TATTATTGAA GCATTTATCA GGGTTATTGT 7980
CTCATGAGCG GATACATATT TGAATGTATT TAGAAAAATA AACAAATAGG GGTTCCGCGC 8040
ACATTTCCCC GAAAAGTGCC ACCTGACGTC TAAGAAACCA TTATTATCAT GACATTAACC 8100
TATAAAAATA GGCGTATCAC GAGGCCCTTT CGTCTTCAAG 8140

## Description

The present invention relates to organisms comprising tasty peptides or parts or extracts thereof, to tasty peptides in homogeneous form, their production via recombinant DNA technology and their use in modifying, enhancing and masking taste impressions.
Most flavors consist of a volatile and a non-volatile part. The non-volatile part being the body of a flavor is perceived in the mouth whereas odorants from the volatile part evaporate into the nasal cavity where they are registered at the olfactory epithelium. There are 4 basic tastes being salty, sour, sweet, bitter and some persons also include umami (Japanese and means: this is good, e.g., glutamate).
Molecules of the non-volatile part have a rather high vapor pressure not allowing them to evaporate under normal atmospheric pressure. These include salts, sugars and other sweeteners, fats (such as butter giving mouthfeel), proteins, peptides and amino acids. The taste of amino acids has been extensively studied mainly being bitter or sweet [Nishimura and Kato, Food Reviews International 4, 175 - 194 (1988)]. A special role is attributed to monosodium glutamate (MSG) which enhances flavor intensity. Several small di- or tri-peptides have been synthesized and evaluated on their taste [Nishimura and Kato, supra]. In such studies peptides have been shown to act as taste enhancers, modifiers, potentiators and masking compounds. Separation of various fish, cheese and meat products into several non-volatile fractions allowed to characterize important tasty peptides. These peptides were naturally formed by proteases (present in the food material) digesting proteins such as casein in milk. In particular, an octapeptide has been found in beef. Due to its meaty taste and ability to enhance meat flavors it was named beefy meaty peptide (BMP), also referred to as STET (savory taste-enhancing peptide).
Tasty peptides are currently prepared from food proteins such as caseins and lactalbumins (milk), muscle proteins (beef), chicken or glutens (soya or wheat). Upon protease digestion the proteins are split into smaller peptides and amino acids. Depending on the protease specificity different tasty peptides are formed. Their length and amino acid sequence are variable. The peptide length can be from 2 to up to 50 amino acids [Lahl and Braun, Food technology 10, 68 - 71 (1994)].
As protease digestion of food proteins results in a complex mixture of tasty peptides the analysis of their individual taste is rather difficult. In combination these peptides may even act synergistically adding more complexity to the taste contribution of the individual peptide.
Several tasty peptides have been purified and their sequences determined as listed below:

| Tasty peptides | Source | Reference |
|---|---|---|
| KGDEESLA (BMP) | Beef meat | Yamasaki and Maekawa Agric.Biol.Chem. 42, 1761-1765 (1978) |
| | | Nakata etal., Biosci.Biotech. Biochem. 59, 689 - 693 (1995) |
| Leu-Pro, Val-Pro, Phe-Pro, ..... | Cheese | Mojarro-Guera etal., Journal of Food Science 56, 943-947 (1991) |
| Ala-Pro-Phe-Pro-Glu, ... | Cheese | Kaiser etal., Z. Lebensm. Unters. Forsch. 195, 8 - 14 (1992) |
| Ala-Val-Pro-Tyr-Pro-Gln-Arg ,... | Cheese | Bumberger and Belitz, Z. Lebensm. Unters. Forsch. 197, 14- 19 (1993) |
| Ala-Glu, Asp-Ala, Asp-Gly, | Fish | Noguchi etal., J. Agr. Food Chem. 23, 49 - 53 (1975) |
| Asp-Glu-Ser, Glu-Asp-Glu, | " | |
| Asp-Glu-Gly-Asp, | " | |
| Asp-Glu-Gly-Ser-Asp, ... | " | |

It has now been found that, instead of producing mixtures of different tasty peptides by digestion of proteins, particular tasty peptides can be obtained by transfer of DNA sequences coding for a said particular tasty peptide into organisms and subsequent expression of the particular tasty peptides. The tasty peptides can either be used in combination with the producing organisms or after purification to homogeneity for modifying, enhancing and masking taste impressions.

Thus, in a first aspect of this invention, there are provided organisms comprising tasty peptides or parts or extracts thereof. These organisms include microorganisms, fungi, plants or animals. Specific organisms comprising tasty peptides include food-grade organisms, for example, Lactococcus lactis, Lactobacillus acidophilus, Staphylococcus carnosus, yeasts, fusarium and filamentons fungi, e.g., Aspergillus or Penicillium.

Parts or extracts of organisms comprising tasty peptides include milk of animals or a protease treated preparation thereof.

In a second aspect of this invention, there are provided tasty peptides in homogeneous form.

As used herein, the term "tasty peptides" means peptides which contribute in combination with volatile compounds to the overall impression of a flavor. Generally, tasty peptides are taste active and are able to give, to modify, to enhance and/or to mask the different taste impressions. Tasty peptides can exhibit their effect either alone or in symphony as a mixture with other tasty peptides. The term "tasty peptides" includes tasty peptides from milk, cheese, meat,poultry, fish, soya and cereals. The peptide length can be from 2 to up to 50 amino acids. Examples of such peptides are disclosed, e.g. in Biosci. Biotech. Biochem. 59, 689 - 693 (1995), Journal of Food Science 56, 943 - 947 (1991), Z. Lebensm. Unters. Forschung 195, 8 - 14 (1992), Z. Lebensm. Unters. Forsch. 197, 14 - 19 (1993) and J. Agr. Food Chem. 23, 49 - 53 (1975). Specifically tasty peptides are defined to include the amino acid residues
Lys-Gly-Asp-Glu-Glu-Ser-Leu-Ala (SEQ ID No: 1),
Asp-Lys-Ile-His-Pro-Phe (SEQ ID No: 2), or
Arg-Gly-Pro-Phe-Ile-Ile-Val (SEQ ID No: 3).

The term "homogeneous" means that a particular tasty peptide can be obtained substantially free from other tasty peptides. For purposes of the present invention, tasty peptides composed of more than 95 percent of the same kind are considered substantially free from other tasty peptides.

As used herein, the term "tasty peptides" includes proteins modified deliberately as, for example, by addition of specific affinity and/or signal sequences.

Specific affinity sequences that preferably bind to an affinity carrier material are, for example, sequences containing at least two adjacent histidine residues (see in this respect European Patent No. 282 042). Such sequences bind selectively to nitrilotriacetic acid nickel chelate resins (Hochuli and Döbeli, Biol. Chem. Hoope-Seyler 368, 748 (1987); European Patent No. 253 303). Tasty peptides which contain such a specific sequence can, therefore, be separated selectively from remaining peptides and polypeptides. The specific sequence can be linked either to the C-terminus or the N-terminus of a particular tasty peptide.

A signal sequence means a sequence included at the beginning of the coding sequence of a protein to be expressed on the surface of a cell. This sequence encodes a signal peptide, N-terminal to the mature polypeptide, that directs the host cell to translocate the polypeptide. The term "translocation signal sequence" is also used herein to refer to this sort of signal sequence. Translocation signal sequences can be found associated with a variety of proteins native to eukaryotes and prokaryotes, and are often functional in both types of organisms. Tasty peptides which contain such a signal sequence can, therefore, be efficiently secreted into cell free medium.

The affinity and/or signal sequences can be linked directly or indirectly to a particular tasty peptide. In the case of indirect linking, the affinity and/or signal sequences contain a suitable selective cleavage site via which they are linked to the particular tasty peptide. A preferred selective cleavage site has the amino acid sequence Glu-Ala, which is specifically recognized by the STE 13 diaminopeptidase. Such affinity and/or signal sequences can then be cleaved enzymatically, using methods known in the art.

The term "tasty peptides" further includes repetitive artificial proteins comprising a particular tasty peptide repeatedly. In order to obtain a monomeric form of a particular tasty peptide, the repetitive artificial proteins are cleaved enzymatically. Examples of proteases for cleavage are lysyl amino peptidases (E.C. 3.4.11.15) or alanine carboxypeptidases (E.C. 3.4.17.6). The proteases can either be added following isolation of the repetitive artificial proteins or can be co-expressed in a particular organism yielding expression and processing of the repetitive artificial proteins at the same time. The term "repetitive artificial proteins" includes proteins modified, as for example, by addition of specific affinity and/or signal sequences as hereinbefore described.

There are also provided isolated DNA sequences encoding tasty peptides of the present invention, vectors and expression vectors containing DNA sequences encoding tasty peptides of the present invention, organisms containing such vectors for the production of tasty peptides, and processes for the production of such DNA sequences, recombinant vectors and organisms.

Methods for the expression and purification of the tasty peptides of the present invention are also provided:

DNA sequences encoding tasty peptides can be produced by conventional techniques known in the art. Using the methods described in this application, oligonucleotides are synthesized which, when assembled correctly, form a synthetic DNA sequence coding for a taste peptide of the present invention. The synthetic DNA sequence can be provided with suitable restriction enzyme sites. They can be obtained in accordance with methods well-known in DNA chemistry.

DNA sequences that code for tasty peptides of the present invention can be introduced in any convenient vector in a manner known per se.

For expression of tasty peptides in host organisms, in principle, all vectors which replicate and express DNA sequences encoding tasty peptides in the chosen organism are suitable. Expression vectors suitable for use in prokaryotic cells are mentioned, for example, in the textbook "Molecular Cloning - A Laboratory Manual", Cold Spring Harbor Laboratory (1982), of Maniatis et al. Examples of other vectors are plasmids of the pDS family [Bujard et al., Methods in Enzymology, eds. Wu and Grossmann, Academic Press, Inc., Vol. 155, 416-433 (1987)].

Such prokaryotic expression vectors which contain the DNA sequences coding for the tasty peptides operatively linked with an expression control sequence can be incorporated using conventional methods into any suitable prokaryotic cell. The selection of a suitable prokaryotic cell is determined by different factors which are well-known in the art. Thus, for example, compatibility with the chosen vector, toxicity of the expression product, expression characteristics, necessary biological safety precautions and costs play a role and a compromise between all of these factors must be found.

Suitable prokaryotic organisms include gram-negative and gram-positive bacteria, for example, E. coli and B. subtilis strains. Examples of prokaryotic organisms are E. coli strain M15 (described as strain OZ 291 by Villarejo et al. in J. Bacteriol. 120, 466-474 [1974] and E. coli W3110 [ATCC No. 27325]). In addition to the aforementioned E. coli strains, however, other generally accessible E. coli strains such as E. coli 294 (ATCC No. 31446) and E. coli RR1 (ATCC No. 31343) can also be used. Especially preferred E. coli cells of the present invention are E. coli DH 5α (Gibco BRL).

For expression of the tasty peptides in E. coli and for secretion of the tasty peptides into cell free medium expression vectors can be utilized including a promoter and a DNA sequence encoding a tasty peptide containing a specific affinity and/or signal sequence of E. coli.

Preferably, an E. coli expression vector comprises:
(a) a promoter sequence, and
(b) a DNA sequence encoding a tasty peptide containing the maltose binding protein of E. coli as specific affinity and signal sequence.

The Lactococcus lactis vector system can also be used for the production of tasty peptides of the present invention (for review see Plattneeuw et al., Appl. Environm. Microbiol. 62, 1008-1013 [1996]). Using specific signal sequences the desired tasty peptide can be secreted or produced intracellularly. The peptide can either be produced in homogeneous form or in combination with the host organism.

The Lactobacillus acidophilus vector system can also be used for the production of tasty peptides of the present invention (for review see Lin et al., Appl. Microbiol. Biotechn. 45, 484-489 [1996]). Using specific signal sequences the desired tasty peptide can be secreted or produced intracellularly. The peptide can either be produced in homogeneous form or in combination with the host organism.

The Staphylococcus carnosus vector system can also be used for the production of tasty peptides of the present invention (for review see Brückner and Götz, System. Appl. Microbiol. 18, 510-516 [1995]). Using specific signal sequences the desired tasty peptide can be secreted or produced intracellularly. The peptide can either be produced in homogeneous form or in combination with the host organism.

In a preferred embodiment of the present invention yeast is used as the organism. Expression vectors suitable for use in yeast cells are described in "Guide to yeast genetics and molecular biology", Guthrie and Fink, eds., Methods in Enzymology, Academic Press, Inc., Vol. 194 (1991) and "Gene expression technology", Goeddel, ed., Methods in Enzymology, Academic Press, Inc., Vol. 185 (1991). The preferred yeast vectors of the present invention are vectors of the pNS family, e.g. pNS2 and pNS3. Examples of suitable yeast cells are Saccharomyces cerevisiae, Pichia pastoris, Hansenula polymorpha, Schizosaccharomyces pombe cells. An overview on various yeast expression systems is given by Romanos et al., Yeast, Vol. 8, 423-488 (1992). Especially preferred yeast cells of the present invention are S. cerevisiae DBY746 [ATCC 44773].

The transformation with the yeast expression vectors is carried out as described by Klebe et al., Gene Vol. 25, 333-341 (1983).

For expression of tasty peptides in yeast and for secretion of these peptides into cell free medium expression vectors can be utilized including a promoter, a DNA sequence encoding a tasty peptide and a DNA sequence encoding a signal sequence, upstream to the DNA sequence encoding the tasty peptide.

Preferably, a yeast expression vector comprises:
(a) a promoter sequence,
(b) the tasty peptide coding sequence, and
(c) the α-factor signal protein coding sequence upstream the tasty peptide coding sequence.

The tasty peptides expressed in yeast can be produced in homogeneous form or in combination with the host organism.

The manner in which the expression of the tasty peptides is carried out depends on the chosen expression vector host cell system.

Usually, the prokaryotic host cells which contain a desired expression vector are grown under conditions which are optimal for the growth of the prokaryotic host cells. At the end of the exponential growth, when the increase in cell number per unit time decreases, the expression of the desired tasty peptide is induced, i.e., the DNA coding for the desired tasty peptide is transcribed and the transcribed mRNA is translated. The induction can be carried out by adding an inducer or a derepressor to the growth medium or by altering a physical parameter, e.g., a change in temperature. For example, the expression can be controlled by the lac repressor.

By adding isopropyl-β-D-thiogalactopyranoside (IPTG), the expression control sequence is derepressed and the synthesis of the desired tasty peptide is thereby induced.

The yeast host cells which contain a desired expression vector are grown under conditions which are optimal for the growth of the yeast host cells. The expression of the desired tasty peptide is induced by e.g., phosphate depletion.

The Fusarium vector system can also be used for the production of tasty peptides of the present invention (for review see Royer et al., Bio/Technology 13, 1479-1483 [1995]). Using specific signal sequences the desired tasty peptide can be secreted or produced intracellularly. The peptide can either be produced in homogeneous form or in combination with the host organism.

Filamentous fungi (such as Aspergillus or Penicillium) vector systems can also be used for the production of tasty peptides of the present invention (for review see Berka and Barnett, Biotech. Adv. 7, 127-154 [1989]; Devchand and Gwynne, J. Biotechnol. 17, 3-9, [1991]). Using specific signal sequences the desired tasty peptide can be secreted or produced intracellularly. The peptide can either be produced in homogeneous form or in combination with the host organism.

The baculovirus-insect cell vector system can also be used for the production of the tasty peptides of the present invention (for review see Luclow and Summers, Bio Technology 6, 47-55 [1988]). The tasty peptides produced in insect cells infected with recombinant baculovirus can undergo post-translational processing including N-glycosylation (Smith et al., Proc. Nat. Scad. Sci. USA 82, 8404-8408 [1985]) and O-glycosylation (Thomsen et al., 12. International Herpesvirus Workshop, University of Philadelphia, Pennsylvania).

Plants can also be used as hosts for the production of tasty peptides of the present invention. Transfer of the DNA sequence coding for the tasty peptide may be achieved by a variety of methods (for review see Potrykus and Spangenberg, eds., Gene transfer to plants. A laboratory manual, Springer Verlag, Heidelberg, Germany (1995)), whereby the DNA sequence for the tasty peptide is integrated into the chromosome of the host plant. Over expression of the tasty peptide may be achieved, for example, by transforming a plant host with the DNA sequence coding for the tasty peptide. Examples of plant hosts for the production of tasty peptide include, but are not limited to maize (Zea mays, Ishida et al., Nature Biotechnology 14, 745-750 (1996)), flax (Linum usitatissimum, Dong and Mchughen, Plant Sci. 88 (1), 61-71 (1993)) and soybean (Glycine max, Christou et al., Tibtech 8, 145-151 (1990)).

Transgenic animal vector systems can also be used for the production of tasty peptides of the present invention (for review see Pinkert, Transgenic animal technology: a laboratory handbook, Academic Press, San Diego [1993]). Using specific signal sequences the desired tasty peptide can also be secreted into the milk of the animal (for examples see Drohan et al., J. Cell. Biochemistry 17a, 38-38 [1993]; Lee et al., Appl. Biochem. Biotechnol. 56, 211-222 [1996]) thus allowing the use of the milk or a protease treated preparation thereof as a food and flavor ingredient containing tasty peptides.

The tasty peptides expressed in the organisms as hereinbefore described can be isolated from the host cell medium using standard purification methods. For analytical purposes tasty peptides can be separated by high performance liquid chromatography (HPLC). Preparative separation is best achieved using size exclusion chromatography. In principle, all other chromatography systems used for protein separation can also be applied for peptide separation. These include, e.g., hydrophobic interaction chromatography (HIC), affinity chromatography, ion exchange chromatography and normal and reverse phase HPLC.

Alternatively, as hereinbefore described, the tasty peptides can be used in combination with the host organism or parts or extracts thereof.

The organisms comprising tasty peptides of the present invention or parts or extracts thereof and the tasty peptides of the present invention can be used to modify, to enhance or to mask the different taste impressions. They can be used in the same manner as those currently produced by protease digestion of proteins. They can be worked into food in a manner known per se.

Having now generally described this invention, the same will become better understood by reference to the specific examples, which are included herein for purpose of illustration only and are not intended to be limiting unless otherwise specified, in connection with the following figures:
- Fig. 1: displays the complete nucleotide sequence of plasmid pNS2.
- Fig. 2: is a schematic drawing of plasmid pNS2.
- Fig. 3: is a schematic drawing of plasmid pNS3.
- Fig. 4: shows an electrospray mass spectrum of BMP. The protonated molecular ion (m/z 848.3) is less abundant than the doubly charged ion (m/z 424.9).

### Example 1

### Expression of beefy meaty peptide (BMP) in E. coli

### A. Construction and transformation of the expression vector

The BMP sequence was cloned into the expression vector pMal-C2 (New England Biolabs (NEB), Beverly, MA, USA). The oligonucleotide sequences A1 and A2 were ordered from a commercial service (Microsynth AG, Balgach, CH) and 0.6 ng of each were mixed in 2 µl H₂O, heated to 80 °C and slowly cooled down to room temperature to allow annealing.
A1: 5' AAGGGTGACGAAGAATCTTTGGCTTAGA-3'
A2: 3'-TTCCCACTGCTTCTTAGAAACCGAATCTTCGA-5'
The annealed oligonucleotides were then ligated with XmnI and HindIII treated pMal-C2 vector using ligase (Gibco, 0.4 U/µl) according to the manufacturer's instructions (NEB). The plasmid now containing the sequence coding for BMP as a fusion protein to maltose binding protein was transformed into *E. coli* DH5α (Gibco BRL).

### B. Purification, cleavage and detection of BMP

A transformed *E. coli* clone was grown in LB medium to an OD₆₀₀ of 0.5 after which 1 mM IPTG was added to induce the production of the fusion protein. The fusion protein was affinity-purified using maltose-agarose (New England Biolabs) starting from sonicated cell supernatant. The fusionprotein was eluted from the column and the so purified fusionprotein was treated with Factor Xa as outlined in the manufacturer's protocol resulting in the cleavage of BMP. BMP was detected by using HPLC chromatography (as given below in the yeast section).

### Example 2

### Expression of beefy meaty peptide (BMP) in yeast

### A. Preparation of a oligonucleotide coding for BMP.

The following oligonucleotides were obtained from Microsynth AG, Balgach, Switzerland:
B1: 5'-TCGAGAAGAGAGAAGCTGAAGCTAAGGGT-3'
B2: 5'-CGTCACCCTTAGCTTCAGCTTCTCTCTTC-3'
B3: 5'-GACGAAGAATCTTTGGCTTGAG-3'
B4: 5'-GATCCTCAAGCCAAAGATTCTT-3'

The oligonucleotides were diluted to a concentration of 200 pmol. 24.8 µl of the oligonucleotide solution was mixed with 3 µl 10x PNK buffer (see Pharmacia's protocol), 1.2 µl 10 mM ATP and 1 µl polynucleotide kinase (Pharmacia, 5.3 U/µl). The mixture was incubated at 37 °C for 45 min after which the kinase was inactivated at 65°C for 10 min.
6 µl of each of the two pairing oligonucleotides (B1 and B2 and B3 and B4) were incubated with 11.2 µl 25 mM MgCl₂, 1.6 µl 0.5 mM Tris-HCl, pH 7.6 and 15.2 µl H₂O at 60 °C for 5 min. Annealing was obtained by slowly cooling to 40°C in 20', then chilled on ice.
For the construction of the oligonucleotide coding for BMP the paired oligonucleotides were mixed and ligated. The reaction mixture consisted of 20 µl of each annealed oligonucleotide, 0.5 µl 1 M DTT, 12.5 µl 10 mM ATP, 3 µl ligase (Gibco, 0.4 U/µl) and water to give a final volume of 100 µl The mixture was then incubated at room temperature for 4 hours.

### B. Preparation of the expression vector

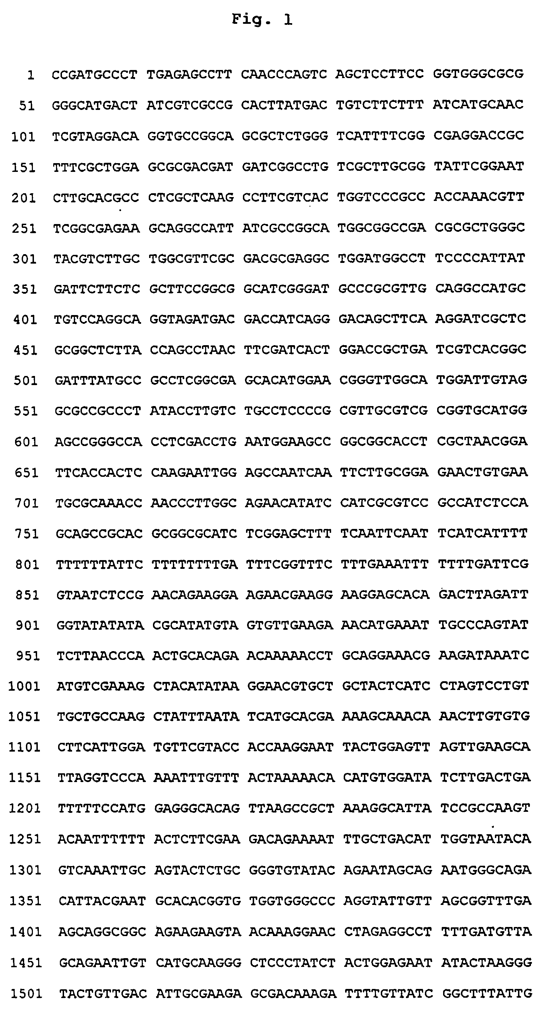

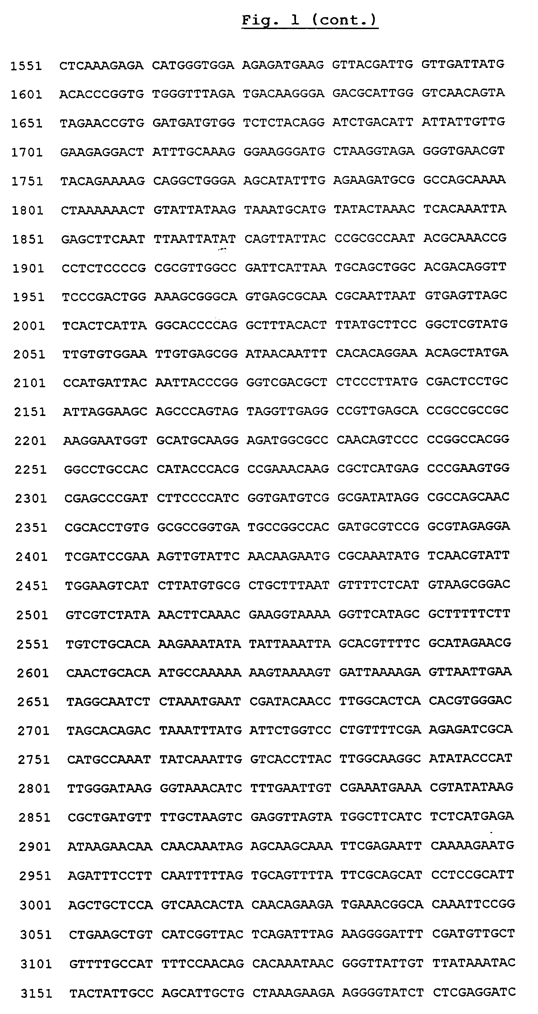

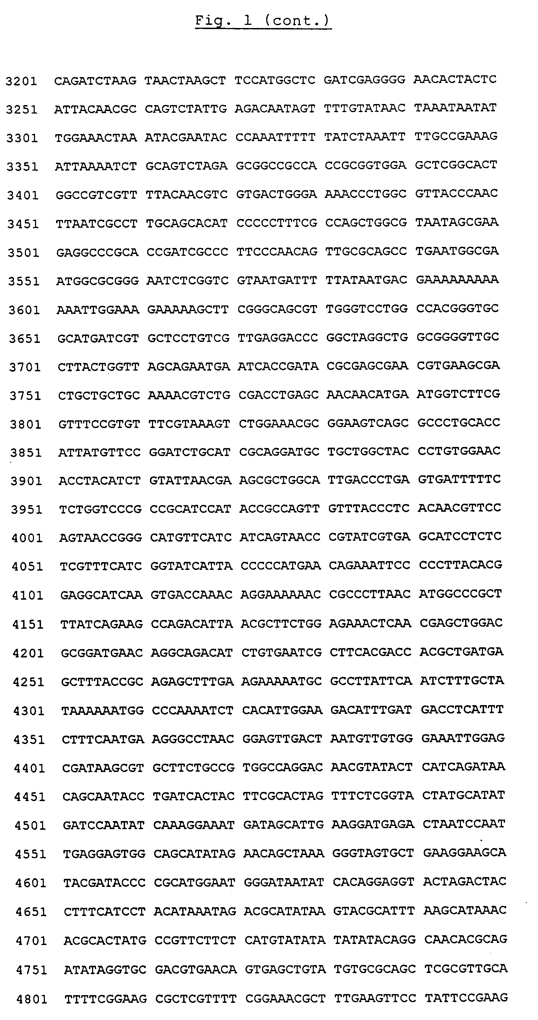

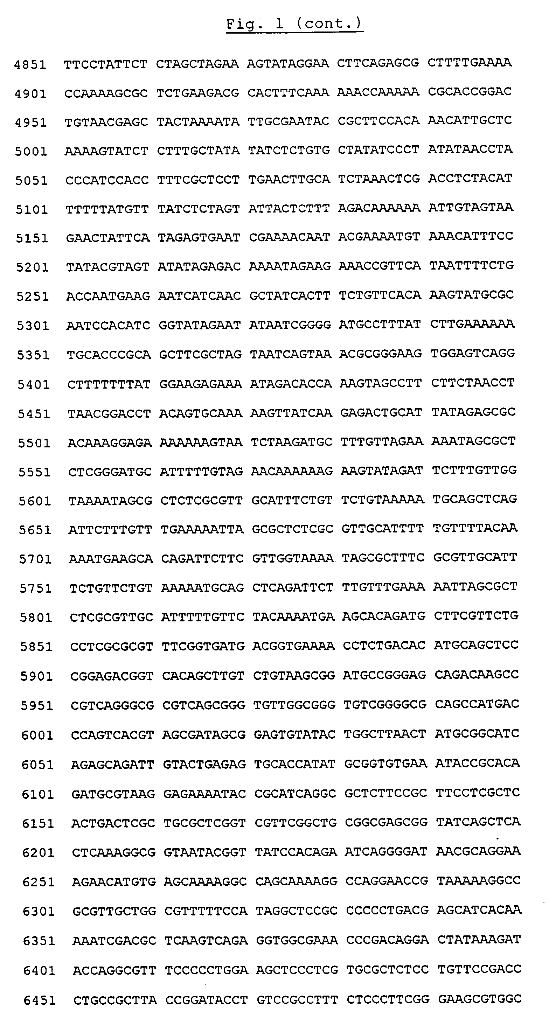

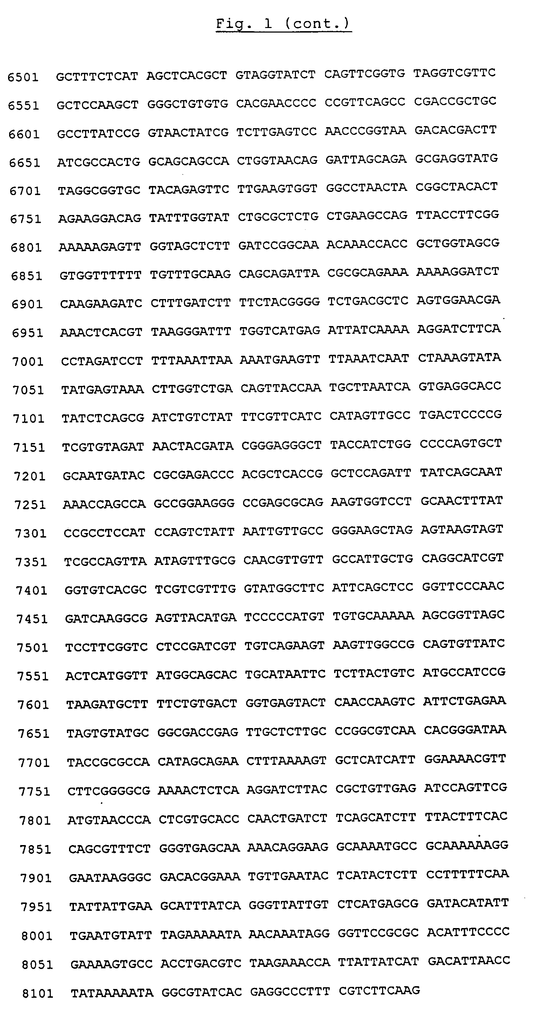

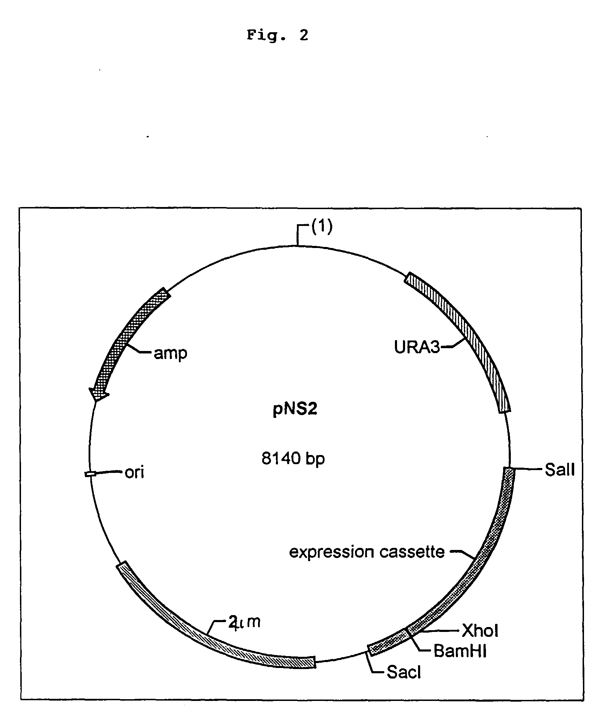

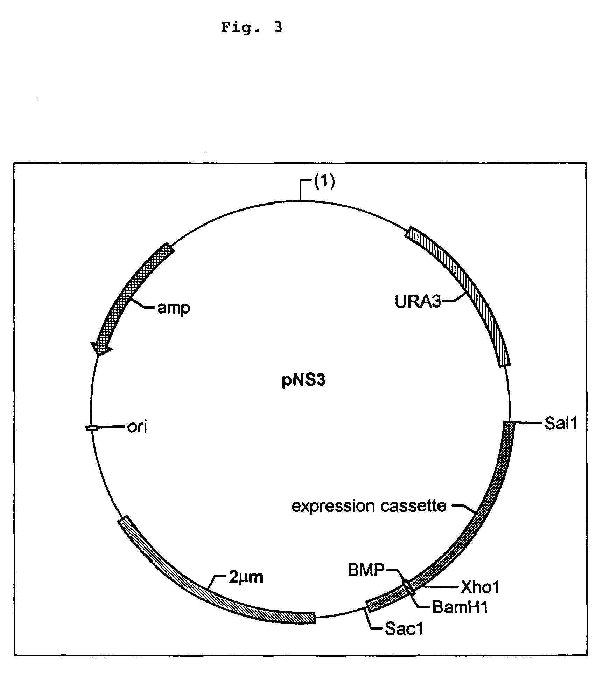

The expression vector pNS2 (Figs. 1 and 2) was cleaved with the restriction enzymes XhoI and BamHI according to the manufacturer's instructions (NEB). About 100 ng of gel-purified, linearized vector were incubated with 1 µl of the oligonucleotides coding for BMP in the presence of ligase (Gibco 0.4 U/µl)4 according to the manufacturer's instruction. The ligated plasmids were then transformed into *E. coli* DH5α (Gibco BRL) from which the plasmid DNA was isolated using Qiagen Plasmid Midi Kit (Qiagen, Germany). The oligonucleotide sequence was determined at a commercial sequencing center (Microsynth AG, Balgach). Plasmids with the oligonucleotide coding for BMP were isolated and used for transformation of the yeast strains. These plasmids were designated pNS3 (Fig. 3).

### C. Transformation of S. cerevisiae

5 µg of vector pNS3 were transformed into *S. cerevisiae* DBY746 (ATCC 44773) as described by Klebe et. al. (supra). The transformed yeast cells were plated onto suitable selective media (SD medium supplemented with the amino acids histidine and leucine, see Sherman in "Guide to yeast genetics and molecular biology", Guthrie and Fink, eds., Methods in Enzymology, Academic Press, Inc., Vol. 194, 3-21 (1991) for description of the medium) and grown for 4 days at 30°C.

### D. Expression of the BMP peptide

Colonies grown on the selective agar media were grown in liquid SD medium supplemented with the above amino acids. Induction was achieved by washing the cells and resuspending them in D3 minimal medium not containing any phosphate when culture densities reached an absorption of 2.0 at 600 nm. An alternative was adding a defined amount of phosphate to the culture medium in order that the cells grow into phosphate depletion and induction occurs. The peptide was intracellularly generated as a fusion protein to the α-factor signal protein. Upon secretion the peptide was cleaved by signal proteases releasing the designed peptide into the medium.

### E. Detection of the peptide by HPLC

Supernatants from the fermentation were separated by reverse phase HPLC using a 4.6 x 250 mm C₈ column (Brownlee column, Applied Biosystems) with a flow rate of 1 ml min⁻¹ and a linear gradient from 0 - 100% in 15 min using water supplemented with 0.1% TFA as buffer A and 80% acetonitrile supplemented with 0.1% TFA as buffer B. The absorbance was read at 202 nm, 214 or 222 nm, respectively. The retention times of the produced peptides were compared with the ones from pure, chemically synthesized BMP.

### F. Detection by LC-MS

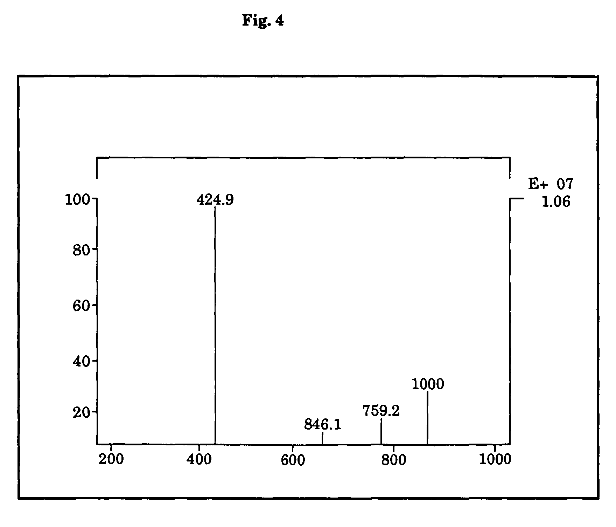

To identify the BMP molecule unambigously, an MS instrument was coupled to the HPLC system. BMP-samples were first analyzed by liquid chromatography (as described above) combined with mass spectrometry (ESI-LC/MS) on a Finnigan MAT SSQ 710C mass spectrometer using electrospray ionization (ESI). A post-column flow split was set up to produce a ratio of 1:5, i.e. a flow of 200µl min⁻¹ was introduced into the ion source. A spray voltage of 4500 V was applied to the electrospray needle. The temperature of the heated capillary was held at 210°C and a mass range from m/z 200-1200 was scanned in 1.5 sec.
The m/z spectra (Fig. 4) obtained from the microbially produced BMP was comparable to the one obtained from synthetic material.

### Example 3

### Expression of BMP as tandem repeats in yeast

An oligonucleotide sequence coding for several tandem repeats of BMP was constructed to enhance the production levels of BMP. Each repeat was preceded by a short spacer peptide (Lys-Arg-Glu-Ala-Glu-Ala) which was cleaved off during secretion. The cloning was done similarily to Example 2. 6 µl of the pairing oligonucleotides (B1 and B2; Example 2) were mixed with 6-60 µl of the pairing oligonucleotides (B5 and B6). The oligonucleotides were annealed and ligated as in Example 2. By varying the concentration of the oligonucleotides B5 and B6 the average number of repeats could be controlled.
B5: 5'-GACGAAGAATCTTTGGCTAAGAGAGAAGCTGAAGCTAAGGGT-3'
B6: 3'-CGTCACCCTTAGCTTCAGCTTCTCTCTTAGCCAAAGATTCTT-5'
To this ligation mixture, 6 µl of the pairing oligonucleotides B3 and B4 (Example 2) were added. The ligated fragments obtained were then ligated into the expression vector pNS2 and the plasmids obtained were transformed into E. coli DH5α as described in Example 2. The number of tandem repeats was determined by estimating the size of a PCR-fragment using primer B1 and the PCR-primer given below. Each repeat accounted to an extra 42 bp in size.
PCR-primer: 5'-TAGACTGGCGTTGTAATG-3'
Plasmids with 2-5 tandem repeats were transformed into S. cerevisiae DBY746. Expression of the BMP tandem peptide was done as described in Example 2. Upon secretion the peptides put in tandem were cleaved resulting in the release of BMP into the medium. The formation of the single BMP units was confirmed by HPLC and LC-MS as done in Example 2.

### Example 4

### Expression of a repetitive artificial proteins comprising BMP repeatedly

A DNA sequence coding for BMP is repeated several times forming concatemeric units. The two following oligonucleotides are annealed as described above:
C3: 5'-GACGAAGAATCTTTGGCTAAGGGT-3'
C4: 3'-TTCTTAGAAACCGATTCCCACTGC-5'
Concatemers are linked together by the addition of ligase (Gibco). Selection for long concatemeric forms (> 600 bp) is done by separation on a polyacrylamide gel (Sambrook et al, supra). The fragments eluted from the gel are then ligated with the two fragments given below, which are obtained by annealing of the respective oligonucleotides.
C1: 5'-TCGAGAAGAGAGAAGCTGAAGCTAAGGGT-3'
C2: 3'-CTTCTCTCTTCGACTTCGATTCCCACTGC-5'
C5: 5'-GACGAAGAATCTTTGGCTTGAG-3'
C6: 3'-TTCTTAGAAACCGAACTCCTAG-3'
The ligation product is then inserted into the yeast expression vector pNS2 previously cleaved with the restriction enzymes XhoI and BamHI. The ligation mixture is transformed into *E. coli* . The plasmid is isolated and transformed into *S. cerevisiae* as described above.
Cultivation and induction of the yeast is performed as described above. The protein is secreted into the medium. In order to obtain the monomeric form of the BMP the repetitive artificial protein is treated by proteases cleaving the Ala-Lys amino acid bond. Possible proteases are lysyl aminopeptidases (E.C. 3.4.11.15) or alanine carboxypeptidases (E.C. 3.4.17.6). The protease is either added after the fermentation or is co-expressed in the yeast strain yielding production and processing of the repetitive artificial protein at the same time.

### Example 5

### Expression of cheese tasty peptide

The cheese peptide DKIHPF (SEQ ID No. 2) was expressed in the same way as that described for BMP. The two following oligonucleotides were annealed and ligated into expression vector pNS2:
5'-TCGAGAAGAGAGAAGCTGAAGCTGACAAGATTCACCCATTCTGAG
3'-CTTCTCTCTTCGACTTCGACTGTTCTAAGTGGGTAAGACTCCTAG-5'
The oligonucleotide fragment was inserted into the XhoI and BamHI cleaved vector pNS2. Transformation, induction and production of peptide was done as described in Example 2. The peptide was detected in a similar manner as the BMP using HPLC or LC-MS. In order to increase the production yield, this peptide is obtained from a tandem repeat sequence or is produced as a repetitive artificial protein as shown in Example 4.

### Example 6

### Expression of peptides with bitter taste

The bitter peptide RGPFPIIV (SEQ ID No. 3) was expressed in the same way as that described for BMP. The following oligonucleotides were annealed and ligated into expression vector pNS2: 
5'-TCGAGAAGAGAGAAGCTGAAGCTAGAGGTCCATTCCCAATTATTGTTTGAG-3'
3'-CTTCTCTCTTCGACTTCGATCTCCAGGTAAGGGTTAATAACAAACTCCTAG-5'
 The oligonucleotide fragment was inserted into the XhoI and BamHI cleaved vector pNS2. Transformation, induction and production of peptide was done as described in Example 2. The peptide was detected in a similar manner as the BMP using HPLC or LC-MS. In order to increase the production yield, this peptide is obtained from a tandem repeat sequence or is produced as a repetitive artificial protein as shown in Example 4.

## Claims

1. Tasty peptides in homogeneous form.

2. A tasty peptide according to claim 1 having a length from 2 to up to 50 amino acids.

3. A tasty peptide according to claim 1 or 2 comprising the amino acid residues SEQ ID No: 1, SEQ ID No: 2 or SEQ ID No: 3.

4. An isolated DNA sequence encoding a tasty peptide as claimed in claims 1 to 3.

5. A vector comprising a DNA sequence as claimed in claim 4.

6. A vector as claimed in claim 5 capable of directing expression in insects, microorganisms, fungi, plants or animals.

7. Organisms transformed with a vector as claimed in claims 5 and 6.

8. The organism of claim 7 which is a yeast.

9. Organisms comprising tasty peptides or parts or extracts thereof.

10. A method for producing a tasty peptide as claimed in claims 1 to 3 comprising cultivating an organism as claimed in claims 7 and 8 in a suitable medium and optionally isolating said protein.

11. Tasty peptides whenever prepared by a process as claimed in claim 10.

12. The use of a tasty peptide as claimed in claims 1 to 3 to give, to modify, to enhance or to mask different taste impressions.

13. The use of an organism as claimed in 7 and 8 to modify, to enhance or to mask different taste impressions.

14. Food comprising tasty peptides prepared by the process of claim 10.
